# FASCICULE DE BREVET EUROPEEN

(11) **EP 1 478 767 B1**
(45) Date de publication et mention de la délivrance du brevet: **29.03.2006**
(21) Numéro de dépôt: 03743921.3
(22) Date de dépôt: 11.03.2003
(51) Int. Cl.: C12Q 1/04, C12Q 1/00, G01N 33/569

(54) **METHODE ET DIAGNOSTIC SEROLOGIQUE IN VITRO DES ENDOCARTIDES INFECTIEUSES**
SEROLOGISCHES IN-VITRO-VERFAHREN ZUR DIAGNOSE VON INFEKTIÖSER ENDOKARDITIS
IN VITRO SEROLOGICAL METHOD OF DIAGNOSING INFECTIVE ENDOCARDITIS

(30) Priorité: 12.03.2002 FR 0203050
(43) Date de publication de la demande: 24.11.2004
(73) Titulaire: Université de la Méditerranée (Aix-Marseille II), 13284 Marseille Cédex 08 (FR)
(72) Inventeur: RAOULT, Didier, F-13008 Marseille (FR)
(74) Mandataire: Domange, Maxime
(86) Numéro de dépôt international: PCT/FR2003/000783
(87) Numéro de publication internationale: WO 2003/076941

(56) Documents cités:
- FR-A- 2 791 357
- FR-A- 2 802 213
- LA SCOLA B ET AL: "Serological cross-reactions between Bartonella quintana, Bartonella henselae, and Coxiella burnetii" JOURNAL OF CLINICAL MICROBIOLOGY 1996 UNITED STATES, vol. 34, no. 9, 1996, pages 2270-2274, XP002233227 ISSN: 0095-1137
- BROUQUI P ET AL: "Endocarditis due to rare and fastidious bacteria." CLINICAL MICROBIOLOGY REVIEWS, vol. 14, no. 1, janvier 2001 (2001-01), pages 177-207, XP002233228 ISSN: 0893-8512
- BRUNEVAL P ET AL: "Detection of fastidious bacteria in cardiac valves in cases of blood culture negative endocarditis." JOURNAL OF CLINICAL PATHOLOGY (LONDON), vol. 54, no. 3, mars 2001 (2001-03), pages 238-240, XP008014473 ISSN: 0021-9746
- DATABASE MEDLINE [en ligne] US NATIONAL LIBRARY OF MEDICINE (NLM), BETHESDA, MD, US; juin 2002 (2002-06) HOUPIKIAN PIERRE ET AL: "Diagnostic methods current best practices and guidelines for identification of difficult-to-culture pathogens in infective endocarditis." Database accession no. NLM12092478 XP002233229 & INFECTIOUS DISEASE CLINICS OF NORTH AMERICA. UNITED STATES JUN 2002, vol. 16, no. 2, juin 2002 (2002-06), pages 377-392, x, ISSN: 0891-5520
- DATABASE EMBASE [en ligne] ELSEVIER SCIENCE PUBLISHERS, AMSTERDAM, NL; 2003 HOUPIKIAN P ET AL: "Western immunoblotting for Bartonella endocarditis" Database accession no. EMB-2003049547 XP002233230 & CLINICAL AND DIAGNOSTIC LABORATORY IMMUNOLOGY 2003 UNITED STATES, vol. 10, no. 1, 2003, pages 95-102, ISSN: 1071-412X

## Description

La présente invention concerne une méthode de diagnostic sérologique *in vitro* des endocardites infectieuses à hémoculture négative chez un patient présentant une endocardite, et plus particulièrement une infection par l'un des deux germes les plus fréquents dans les endocardites à hémoculture négative, à savoir *Coxiella burnetii* et *Bartonella sp.*

La présente invention concerne également une trousse de diagnostic utile pour la mise en oeuvre de ladite méthode de diagnostic sérologie in vitro.

Les bactéries du genre *Bartonella sp* et *Coxiella burnetii* sont impliquées dans un grand nombre de maladies. Les différentes formes de maladies liées à la bactérie *Bartonella* sont les maladies suivantes : maladie des griffes du chat, angiomatose bacillaire, fièvre des tranchées, peliose hépatique et endocardite.

Les maladies dues à des infections causées par une bactérie du genre *Coxiella* se définissent comme la fièvre Q et peuvent se traduire par :
- des infections aiguës : fièvre, hépatite, pneumonie, myocardite, péricardite ou méningite,
- des infections chroniques : endocardite, péricardite, infection vasculaire, ostéite, tumeur pulmonaire ou avortements à répétition.

Les endocardites résultent de l'infection bactérienne d'une valve cardiaque. Les patients souffrant d'endocardite ont donc une lésion valvulaire, identifiable par l'interrogatoire (maladie connue du patient) par l'examen médical (présence d'un souffle cardiaque) ou radiologique (echocardiographie). Le diagnostic des endocardites à germes banals est basé sur l'hémoculture. Il existe des endocardites à hémoculture négative dont la cause peut être (mais pas nécessairement) une infection à bactéries *Bartonella* ou *Coxiella.*

D'autre part, certains porteurs d'une fièvre Q aiguë ou d'une infection ne sont pas atteints d'endocardite.

Actuellement, lorsqu'on veut diagnostiquer une endocardite à hémoculture négative, en particulier une endocardite liée à une infection par *Coxiella burnetii* ou par une bactérie du genre *Bartonella,* il est nécessaire d'avoir recours à des sérologies spécifiques pour chacune de ces deux types de bactéries. [(Raoult D. et al., Ann. lntern. Med., 1996, 125 :646-652 et Drancourt M., et al., New Engl J Med. 1995, 332 :419-423)].et la bactérie Bartonella quintana déposée à l'ATCC sous le numéro 49193.

Actuellement, le diagnostic des maladies liées à une infection par la bactérie *Coxiella burnetii* se fait par un dosage parallèle des anticorps contre les antigènes de phase I et des anticorps de phase II qui sont les deux formes distinctes spécifiques de la bactérie *Coxiella burnetii.* Les endocardites s'accompagnent usuellement d'une augmentation importante des anticorps dirigés contre la phase I en même temps que ceux dirigés contre la phase II. Le sérum à tester est soumis au dépistage pour les deux formes phase I et phase II de la bactérie *Coxiella burnetii* séparément dans des kits de diagnostic différents. En outre, ce dépistage se fait en détectant l'ensemble des immunoglobulines (lgG, IgM et IgA) ou en testant séparément les immunoglobulines G, M et A. La sérologie peut être prescrite pour des infections aiguës, à savoir fièvre, hépatite, pneumonie, myocardite, péricardite, méningite, ou chroniques, à savoir endocardites, péricardite, infections vasculaires, ostéite, tumeur pulmonaire, avortements à répétition. Quand les résultats sont positifs, on réalise une évaluation quantitative des anticorps à partir de tests impliquant des dilutions croissantes de chaque type d'immunoglobuline à des dilutions allant du 1/50ème jusqu'au 1/3200ème afin de déterminer le titre précis.

De même, actuellement, les méthodes de diagnostic de maladies bactériennes avec des bactéries du genre *Bartonella* sont réalisées avec des antigènes qui sont soit la bactérie *Bartonella henselae* soit *Bartonella quintana* qui sont testées à des dilutions successives en détectant la présence d'immunoglobuline totale ou dans certains cas IgGM. Dans ce cas, on prescrit le même examen sérologique pour les différentes formes d'infection à *Bartonella,* à savoir maladie des griffes du chat, engiomatose bacillaire, bactériémie, fièvre des tranchées, pelliose hépatique et endocardite.

On sait qu'environ 80 % des endocardites à hémoculture négative en France sont causées par ces bactéries *(Coxiella burnetii* à raison d'environ 50 % et les bactéries *Bartonella* à raison d'environ 30 %).

Les traitements des endocardites sont bien évidemment très différents selon la cause infectieuse.

Il n'existe pas à l'heure actuelle de kit de diagnostic pour le diagnostic sérologique spécifique des endocardites, notamment pour détecter les endocardites spécifiques d'infections bactériennes à *Coxiella burnetii* ou *Bartonella.*

Le but de la présente invention est de fournir une méthode de diagnostic in vitro d'usage simple, rapide, impliquant un unique test pour déterminer la présence ou non d'une endocardite à *Bartonella sp* ou à *Coxiella burnetii* chez les malades atteints d'une affection des valves cardiaques.

Pour ce faire, la présente invention fournit une méthode de diagnostic sérologique *in vitro* des endocardites infectieuses à hémoculture négative causées par les bactéries *Coxiella burnetii* et *Bartonella sp* chez un malade atteint d'une affection des valves cardiaques, caractérisée en ce qu'on réalise un dosage des anticorps de type IgG dirigés contre la bactérie *Coxiella burnetii* de phase I et des anticorps de type IgG dirigés contre la bactérie *Bartonella sp,* sur un même sérum d'un dit malade, et on détermine si le titre desdits anticorps IgG est supérieur ou égal à 1/800^{ème}, c'est à dire que l'on vérifie si l'on peut détecter la présence desdits anticorps IgG dans un sérum de patient dilué au 1/800^{ème}.

La présente invention fournit un test rapide et simple en testant une dilution unique d'un sérum permettant non seulement de reconnaître que le patient a des anticorps, mais encore qu'il présente des anticorps à un taux élevé donné permettant d'identifier une dite endocardite. La présente invention permet plus particulièrement de déterminer sur un sérum unique, notamment par une technique d'immunofluorescence indirecte, en utilisant une seule dilution, une infection par l'un des deux germes les plus fréquents dans les endocardites à hémoculture négative, à savoir *Coxiella burnetii* et *Bartonella sp,* chez un patient présentant une affection des valves cardiaques décelée cliniquement ou par échocardiographie. La présente invention a en effet permis de déterminer une concentration unique des anticorps anti IgG de la bactérie *Coxiella burnetii* de phase I et d'une bactérie *Bartonella sp* à partir de laquelle dans un échantillon particulier d'un patient présentant des endocardites, le résultat peut conclure qu'il existe ou non une endocardite à l'un des deux germes.

Dans un mode de réalisation particulier et avantageux, on réalise un dosage dans lequel on dépose les deux dits antigènes-bactéries sur un support solide, et on met en oeuvre une seule dilution de sérum à tester dans lequel on a ajouté une immunoglobuline anti IgG en excès, de préférence marquée, que l'on fait réagir avec ledit support solide.

Selon la présente invention, on utilise uniquement la bactérie *Coxiella burnetii* de phase I, car on a observé qu'un taux élevé d'anticorps contre la phase I, notamment au delà de 1/800ème n'apparaît que dans les formes chroniques d'infection à *Coxiella burneti* incluant les endocardites. De même, ces taux d'anticorps supérieurs à 1/800ème sont spécifiques d'une infection chronique appartenant à une bactérie du genre *Bartonella* incluant les endocardites.

En conséquence, pour des sujets souffrant d'une affection endocardiaque, la présence d'anticorps contre l'un de ces deux antigènes a un titre supérieur à 1/800ème est nécessairement liée à une infection par l'une de ces deux bactéries, car la seule affection chronique liée à ces deux germes et causant des affections au niveau cardiaque est une affection du type endocardite.

Par ailleurs; selon la présente invention, il a été observé que la détection d'anticorps entre la bactérie *Coxiella burnetii* ou la bactérie *Bartonella* du type IgM pouvait être due à la présence d'un facteur rhumatoïde. Les facteur rhumatoïdes sont des immunoglobuline de type IgM anti IgG dans la plupart des cas, et risque de donner des faux positifs quand il existe des taux faibles d'IgG chez des patients souffrant d'endocardite à d'autres germes, alors que les patients présentant une endocardite ont souvent des facteurs rhumatoïdes. C'est pourquoi, selon la présente invention, on détecte spécifiquement des anticorps dirigés contre les lgG (et non les IgM ou les immunoglobulines totales), de façon à éliminer les faux positifs dus à des taux faibles d'IgG associés à des facteurs rhumatoïdes (qui sont des IgM anti-IgG).

Le test selon la présente invention représente une simplification et un gain de temps considérable par rapport aux techniques de dépistage et de diagnostic actuellement mis en oeuvre. En effet, on en teste pas de dilution de dépistage et on ne fait qu'un test par sérum. On a déterminé une dilution de 1/800^{ème} qui représente le meilleur rapport sensibilité-spécificité appliqué aux deux bactéries, qui permet de regrouper sur un seul test la détection des bactéries en cause. Selon la présente invention, on réalise une approche originale de diagnostic par syndrome et non pas par maladie bactérienne comme antérieurement, en limitant la question posée sérologiquement au problème de diagnostic de l'endocardite et en remplaçant l'ensemble des techniques sérologiques ultérieurement mises en oeuvre, par une seule technique qui réalise à la fois le dépistage et le diagnostic sur un titre unique.

Dans un mode de réalisation avantageux, on réalise un dosage dans lequel :
1. on dépose les deux dites bactéries sur un support solide, et
2. on fait réagir lesdites bactéries avec un sérum dudit malade, dilué au 1/800^{ème} dans lequel on a ajouté une immunoglobuline anti IgG humaine, de préférence marquée, et
3. on vérifie si lesdites immunoglobulines anti IgG humaines, de préférence marquées, se fixent sur ledit support solide.

En d'autres termes, si après lavage dudit support solide on détecte desdites immunoglobulines anti IgG marquées fixées à celui-ci, leur fixation sur ledit support solide n'a pu se faire que par l'intermédiaire des anticorps IgG (Ac) dressés contre ladite bactérie (Ag) en formant un complexe Ag-Ac-Ig anti IgG.

Dans un mode préféré de réalisation, ladite bactérie *Bartonella henselae ou Bartonella quintana.*

Dans un mode de réalisation particulier, on dépose sur ledit support solide les bactéries suivantes :
- une bactérie *Coxiella burnetii* de phase I,
- une bactérie *Bartonella quintana.*

Des bactéries *Coxiella burnetii* de phase I et *Bartonella* sont accessibles au public par diverses sources. Elles ont été décrites dans la littérature [Tissot Dupont H., et al. Clin. Diag. Lab. Immunol., 1994, 1 :189-196], et plusieurs ont été déposées dans la collection de dépôt de l'ATCC, notamment la bactérie *Coxiella burnetii* de phase I déposée à l'ATCC sous le numéro VR615.

Comme support solide, on peut utiliser tout dispositif adapté à la manipulation de suspensions cellulaires et bactériennes et notamment des tubes, des lames de verre, des tubes Bijoux ou des plaques rigides de microtitrage en polyéthylène, polystyrène, chlorure de polyvinyle ou nitrocellulose comportant des micropuits, les lames de verre étant préférées.

Comme type de marquage de l'immunoglobuline anti-humaine, on utilise donc avantageusement un marquage enzymatique, radioactif ou fluorescent, ce dernier type de marquage étant préféré.

L'expression « marquage fluorescent » signifie que l'anticorps a été rendu fluorescent par couplage ou complexation avec un agent fluorescent approprié tel que l'iso(thio)cyanate de fluorescéine.

L'expression « marquage radioactif » signifie que l'anticorps porte un isotope radioactif permettant de le doser par comptage de la radioactivité qui lui est associée, l'isotope pouvant être porté soit sur un élément de la structure de l'anticorps, par exemple les résidus de tyrosine constitutifs, soit sur un radical approprié qui lui a été fixé.

L'expression « marquage enzymatique » signifie que l'anticorps spécifique est couplé ou complexé à une enzyme qui, associée à l'emploi de réactifs appropriés, permet une mesure quantitative de cet anticorps spécifique.

Le substrat et les réactifs sont choisis de sorte que le produit final de la réaction ou de la séquence de réactions provoquée par l'enzyme et mettant en oeuvre ces substances soit :
- ou bien une substance colorée ou fluorescente qui diffuse dans le milieu liquide environnant l'échantillon testé et qui fait l'objet, soit de la mesure finale spectrophotométrique ou fluorimétrique, respectivement, soit d'une évaluation à l'oeil ; éventuellement par rapport à une gamme de teintes étalonnées,
- ou bien une substance colorée insoluble qui se dépose sur l'échantillon testé et qui peut faire l'objet, soit d'une mesure photométrique par réflexion, soit d'une évaluation à l'oeil, éventuellement par rapport à une gamme de teintes étalonnées.

Lorsque l'on utilise une immunoglobuline rendue fluorescente, la fluorescence associée à l'échantillon testé est lue directement sur un appareil approprié.

Lorsqu'on utilise une sonde radioactive, comme par exemple l'iode 125, la radioactivité associée à l'échantillon testé est complétée dans un compteur gamma selon toute modalité appropriée et par exemple après solubilisation des cellules par une solution alcaline (par exemple une solution de soude) et récupération de la solution contenant la radioactivité à l'aide d'un tampon absorbant.

Lorsque l'on utilise une enzyme sur l'anticorps spécifique, l'apparition d'un produit coloré ou fluorescent est obtenue en ajoutant une solution contenant le substrat de l'enzyme et un ou plusieurs réactifs auxiliaires permettant d'obtenir finalement comme produit de réaction, soit un produit coloré soluble dans le milieu, soit un produit coloré insoluble, soit un produit fluorescent soluble, comme cela a été expliqué précédemment. On mesure ensuite le signal lumineux provenant des échantillons ainsi traités, à l'aide de l'appareillage adapté à chaque cas : photomètre en transmission, ou en réflexion ou fluorimètre respectivement. Alternativement, on peut aussi évaluer à l'oeil la coloration obtenue, en s'aidant éventuellement d'une gamme de solutions colorées étalonnées.

En utilisant comme enzyme la phosphatase alcaline, le couplage de cette enzyme avec l'anticorps spécifique est effectué selon la méthode proposée par Boehringer Mannheim-Biochemica. Les substrats préférentiels de cette enzyme sont le paranitrophénylphosphate pour une lecture fluorométrique ou le bromo-5 chloro-4-umbelliféryl phosphate pour une lecture fluorométrique ou le bromo-5 chloro-4 indolyl-6 phosphate pour obtenir un produit de réaction coloré insoluble. On peut de même utiliser comme enzyme la β-galactosidase dont les substrats préférentiels sont l'orthonitrophényl β-D-galactropyranoside ou le méthyl-4 umbelliféryl β-D-galactopyranoside.

Préférentiellement, on peut coupler les anticorps spécifiques à la péroxydase. Dans ce cas ; le procédé de couplage est dérivé de celui décrit par M.B. Wilson et P.K. Nakane in Immunofluorescence and related staining techniques, W. Knapp, K. Kolubar, G. Wicks ed. Elsevier/North Holland. Amsterdam 1978, p. 215-224.

Les réactifs utilisés pour révéler la péroxydase conjuguée aux anticorps spécifiques contiennent de l'eau oxygénée, sustrat de l'enzyme, et un chromogène approprié par exemple de l'orthophénylénediamine ou l'acide azino-2-2' bis (éthyl-3 thiazoline sulfonique-6) ou ABTS pour obtenir un produit final de réaction coloré et soluble dans le milieu ou bien la diamino-3,3' benzidine ou l'amino-3 éthyl-9 carbazole ou le chloro-4 α-naphtol pour obtenir un produit final de réaction insoluble, ou bien l'acide parahydroxyphényl propionique pour obtenir un produit de réaction fluorescent soluble dans le milieu.

Un autre mode de réalisation de l'invention est l'utilisation d'immunoglobuline couplée à l'acétylcholinestérase.

L'acétylcholinestérase est couplée à l'anticorps en utilisant préférentiellement un procédé dérivé de celui décrit dans le brevet français n° 2 550 799 ou un procédé qui comporte schématiquement la préparation de fragments de l'anticorps par une technique connue, la modification de l'enzyme par réaction avec un agent hétérobifonctionnel approprié et enfin le couplage des produits ainsi obtenus. D'autres procédés connus de construction de conjugués immunoenzymatiques peuvent aussi être utilisés dans ce cas.

La révélation de l'activité enzymatique spécifiquement liée à l'antigène reconnu par le conjugué à l'acéthylcholinestérase est réalisée de préférence selon la technique bien connue qui emploie l'acéthylthiocholine comme substrat de l'enzyme et le réactif d'Ellman, ou acide dithio-5,5' nitro-2 benzoïque comme chromogène, selon toute variante adaptée au cas examiné, par exemple celle décrite par Pradelles et al., dans Anal. Chem. 1985, 57 :1170-1173.

Les chromogènes cités sont utilisés tels quels ou sous forme de sels solubles dans l'eau.

D'autres caractéristiques et avantages de la présente invention apparaîtront à la lumière de l'exposé détaillé qui va suivre, qui décrit l'expérience détaillée dans le but de réaliser l'invention, et qui sont donnés à titre purement illustratifs.

### 1 - Les antigènes

*Bartonella quintana* (souche Oklahoma (ATCC n°49193) et souche Marseille (Faculté de Médecine, 27 Boulevard Jean Moulin 13385 Marseille cedex 5 - collection de dépôt de l'Unité des Rickettsies) *Bartonella henselae* (souche Houston (N°ATCC 49882) et souche Marseille (Faculté de Médecine, 27 Boulevard Jean Moulin 13385 Marseille cedex 5 - collection de dépôt de l'Unité des Rickettsies) ont été utilisés comme antigènes pour les Bartonella. L'antigène de *Bartonella quintana* est obtenu comme suit : la bactérie est cultivée en gélose au sang frais à 10%. Il s'agit d'une souche, appelée Oklahoma. Cette souche sert à inoculer des cellules en culture qui sont des cellules endothéliales humaines, en lignée cellulaire appelée ECV. Après 24 heures d'inoculation ces cellules sont récoltées. Les antigènes bénéficient d'une semi-purification, consistant à enlever les débris cellulaires par centrifugation, puis sont fixés à l'azide de sodium, avant d'être utilisés.

Les bactéries ont été cultivées sur une lignée de cellule endothéliales (ECV 304), récoltées, ajustées à 1 mg/ml et utilisées comme antigène directement. Cette production d'antigène a fait l'objet de publications [D. Raoult., H, Tissot-Dupont., M. Enea Mutillod., Clin. Infect. Dis., 1994, 19 :335] [Raoult D., Foumier PE., Drancourt M. et al, Ann. Intern. Med., 1996, 125 :646-652].

Les antigènes de *Coxiella burnetii* étaient produits grâce à la souche Nine Mile (ATCC N°VR615). Pour obtenir de l'antigène de phase I, la souche est injectée dans une souris BalbC par voie intrapéritonéale. Au 7^{ème} jour, la rate de cette souris est récoltée, broyée et inoculée à ces cellules Vero en culture. Une fois que la bactérie s'est multipliée dans les cellules Vero, les bactéries sont récoltées comme déjà publiées et servent à réinoculer d'autres cellules. L'antigène de phase I est obtenu dans les 6 premiers passages en culture sur cellules Vero. Les phases II sont obtenues au-delà du 15^{ème} passage sur cellules Véro. La phase II est un mutant obtenu en culture de la phase I. L'antigène de *Coxiella burnetii* récolté fait l'objet de deux centrifugations successives afin d'obtenir un antigène semi-purifié, ajusté à 1 mg/ml. Cette technique a été décrite [Fournier PE., Marrie TJ., Raoult D. J. Clin. Microbiol., 1998, 36:1823-1834]. Ces antigènes sont déposés au porte-plume sur une lame téflonisée à 10 trous Dynatech®.

### 2 - Les sérums testés

L'inventeur pour pouvoir mettre au point la technique, a sélectionné un certain nombre de sérums de patients ayant présenté une pathologie identifiée. Il s'agit de patients porteurs d'endocardites de la fièvre Q, ou à *Bartonella* et des patients porteurs d'endocardites à d'autres germes, afin d'avoir des contrôles négatifs, mais aussi des patients porteurs d'une fièvre Q aiguë (sans endocardite), des patients porteurs d'une maladie des griffes du chat (infection causée par *Bartonella* mais sans endocardite) et aussi des malades atteints d'autres maladies identifiées (infections à cytomégalovirus, infection à virus Epstein Barr) .

244 sérums ont été testés. Les sérums sélectionnés consistent en 40 sérums de patients venant prélevé en transfusion sanguine et qui ne présentent aucune maladie, 15 sérums de patients présentant une maladie des griffes du chat, présentant un diagnostic réalisé par d'autres moyens (PCR sur le ganglion), 15 patients ayant présenté une fièvre Q aiguë (diagnostiquée par la mise en évidence d'une séroconversion), 30 patients diagnostiqués comme ayant une endocardite de la fièvre Q (diagnostiquée par mise en évidence de la bactérie sur les valves du patient) et les sérums de 20 patients présentant une endocardite à Bartonella (diagnostic fait par mise en évidence par culture ou par PCR sur les valves du patient). Par ailleurs, 99 sérums de patients présentant une endocardite dont l'étiologie n'est pas *Coxiella burnetii* ou *Bartonella* ont été testés de même qu'un panel de patients présentant une infection documentée à une autre maladie (ni une endocardite, ni une infection à *Bartonella* ou à *Coxiella).* De plus, 25 patients présentant une autre maladie ont été testés. Il s'agit de 5 patients présentant une mononucléose infectieuse, 5 patients présentant une infection à Cytomegalovirus, 5 présentant une séroconversion à virus herpès, 5 patients présentant une hépatite B et 5 patients présentant une séroconversion pour le virus HIV, l'agent du SIDA. Parmi les patients présentant une endocardite, 11 présentaient un facteur rhumatoïde détecté par le test au latex. (Rapitex, RF. Dade Behring))

### 3 - Technique sérologique

La technique sérologique réalisée consiste à déposer les 6 antigènes sur une lame *(Bartonella quintana* Marseille, *Bartonella quintana* Oklahoma, *Bartonella henselae* Houston (ATCC N° 49882) et Marseille et *Coxiella burnetii* phase 1 et phase II (Faculté de Médecine, 27 Boulevard Jean Moulin 13385 Marseille cedex 5 - collection de dépôt de l'Unité des Rickettsies).

Les lames sont des lames de verre présentant 20 spots. Ces lames sont dégraissées, l'antigène est déposé à la surface à la plume ou à la micropipette de façon tangentielle à la partie supérieure du cercle pour *Coxiella burnetii,* puis avec une autre pipette à la partie inférieure du même spot sur chaque spot pour *Bartonella quintana.* Une fois l'antigène déposé, il est fixé par une immersion de la lame de dix minutes dans l'acétone. Une fois la lame sèche, la dilution du sérum est déposée,

Des dilutions du sérum ont été testées au 1/400, 1/800, 1/1600 et 1/3200 dans du PBS. Les sérums ainsi dilués dans du PBS ont été déposés, [D. Raoult., H, Tissot-Dupont., M. Enea Mutillod., Clin. Infect. Dis., 1994, 19:335], laissés incuber pendant 30 minutes, lavés trois fois. L'anticorps anti-immunoglobuline humaine a été alors ajouté à la dilution préconisée par le fabricant, à savoir 1/400^{ème}. Celui utilisé (Bio Mérieux) était soit un anticorps anti-immunoglobuline total (contre un mélange d'lgG, IgM, IgA) soit un anticorps spécifiquement contre les IgG. Les lames ont été incubées pendant trente minutes puis lavées trois fois dix minutes dans du PBS, séchées. Une lamelle de verre est déposée sur la lame avec une goutte de glycérine entre la lame et la lamelle et observée en immunofluorescence. Chaque lame comporte un témoin positif et un témoin négatif, et les spots sont lus au microscope à fluorescence et au grossissement multiplié par 400.

### 4 - Résultats

La présence d'une réaction franche contre l'un ou l'autre des antigènes signe que l'endocardite, observée chez le patient qui a été prélevé, est une endocardite au germe présentant la réaction positive. La présence des deux antigènes est justifiée par le fait qu'il existe des réactions croisées entre les deux mais qu'il s'agit généralement d'infections à *Coxiella burnetii* ayant des faibles titres d'anticorps à *Bartonella .* Le fait d'avoir les deux réactions positives peut amener à déclencher un test plus complet avec *Coxiella burnetii.* Enfin il s'agit des deux germes représentant la plus grande partie des endocardites à hémocultures négatives. Dans une série récente de l'unité des rickettsies [Houpikian P. Diagnostic des endocardites à hémocultures négatives. Thèse de Médecine, Marseille, 2001.], 50% de 300 endocardites à hémocultures négatives étaient causées par *Coxiella burnetii* et 30% par *Battonella.*

Les résultats ont montré que la détection d'immunoglobuline totale posait un problème de spécificité. En effet, deux patients présentant une endocardite à germes banals et des facteurs rhumatoïdes présentaient des anticorps au 1/800 contre les *Bartonella.* Deuxièmement, le travail a montré qu'il n'était pas utile de tester à la fois *Coxiella burnetii* phase I et phase II car toutes les endocardites avaient des anticorps contre la phase I à un titre supérieur ou égale à 1/800 alors qu'une seule fièvre Q sans endocardite avait des anticorps supérieurs ou égaux à 1/800 contre la phase I. En revanche, si toutes les endocardites avaient aussi des anticorps contre la phase II à un titre supérieur ou égale à 1/800, 10 des fièvres Q avaient des anticorps supérieurs ou égaux à 1/800 pour la phase II. Il a donc été décidé de ne plus tester la phase II et de ne prendre que la phase I pour le diagnostic des endocardites.

Pour les infections à *Bartonella,* quelle que soit la cause de l'endocardite à *Bartonella (Bartonella henselae, Bartonella quintana)* l'antigène de *Bartonella quintana* Marseille ou Oklahoma permettait à la dilution du sérum au 1/800 d'identifier toutes les causes endocardites. Il y avait 15 endocardites à *Bartonella quintana* et 5 endocardites à *Bartonella henselae* et tout était détecté par les antigènes de *Bartonella quintana.*

Une dilution au 1/1600^{ème} ne détectait que 11 sur 20 des endocardites à *Bartonella.* En revanche, une dilution au 1/800 permettait de détecter toutes les endocardites à *Bartonella.* Toutes les endocardites à *Coxiella bumetii* étaient aussi détectées au 1/800 et au 1/1600. A une dilution de 1/800 aucun des contrôles négatifs n'étaient positifs pour *Bartonella* ou *Coxiella burnetii,* parmi les patients souffrant soit de maladies des griffes du chat soit d'endocardites à d'autres germes soit d'autres maladies. Au total, parmi les 244 sérums testés parmi lesquels 50 d'endocardites à *Coxiella burnetii* ou à *Bartonella henselae* ou *B. quintana.* Une sérologie en immunoflurorescence utilisant seulement les antigènes *Bartonella quintana et Coxiella burnetii* phase I ont permis d'avoir 50 résultats positifs pour les 50 endocardites et 1 résultat positif pour une fièvre Q aiguë.

Si l'on évalue la valeur de la sérologie parmi les endocardites sur 149 cas testés, aucune endocardite à un germe autre que *Bartonella* ou *Coxiella burnetii* n'a été détecté avec cette technique utilisant que les IgG contre *Coxiella burnetii* phase I et *Bartonella quintana.*

## Revendications

1. Méthode de diagnostic sérologique *in vitro* des endocardites infectieuses à hémoculture négative causées par les bactéries *Coxiella burnetii* et *Bartonella sp* chez un malade atteint d'une affection des valves cardiaques, **caractérisée en ce qu'**on réalise un dosage des anticorps de type IgG dirigés contre la bactérie *Coxiella burnetii* de phase I et des anticorps de type IgG dirigés contre la bactérie *Bartonella sp,* sur un même sérum d'un dit malade, et on détermine si le titre desdits anticorps IgG est supérieur ou égal à 1/800^{ème}_{.}

2. Méthode de diagnostic selon la revendication 1, **caractérisée en ce qu'**on réalise un dosage dans lequel :
1. on dépose les deux dites bactéries sur un support solide, et
2. on fait réagir lesdites bactéries avec un sérum dudit malade dilué au 1/800^{éme}, dans lequel on a ajouté une immunoglobuline anti IgG humaine, de préférence marquée, et
3. on vérifie si lesdites immunoglobulines anti IgG humaines, de préférence marquées, se fixent sur ledit support solide.

3. Méthode de diagnostic selon la revendication 1 ou 2, **caractérisée en ce que** ladite bactérie *Bartonella sp* est une bactérie *Bartonella quintana ou Bartonella henselae.*

4. Méthode de diagnostic selon l'une des revendications 1 à 3, **caractérisée en ce qu'**on dépose sur ledit support solide les bactéries suivantes :
- une bactérie *Coxiella burnetii de* phase 1, et
- une bactérie *Bartonella quintana,*

5. Méthode de diagnostic selon l'une des revendications 1 à 4, **caractérisée en ce que** l'immunoglobuline anti IgG humaine comprend un marquage de préférence détectable par fluorescence.

6. Trousse de diagnostic utile pour la mise en oeuvre d'une méthode de diagnostic selon l'une des revendications 1 à 5, **caractérisée en ce qu'**elle comprend :
- un support solide sur lequel ont été déposées comme dites bactéries responsables des endocardites , uniquement les bactéries *Coxiella burnetii* de phase I et une bactérie *Bartonella sp.,* et
- comme solution comprenant une immunoglobuline anti humaine impliquée dans la détection d'anticorps dirigés contre lesdites bactéries, une seule solution comprenant uniquement comme immunoglobuline anti humaine, une immunoglobuline anti IgG.

## Patentansprüche

1. Methode zur serologischen Diagnose in vitro von infektiöser Endokarditis mit negativer Hämokultur, die durch die Bakterien Coxiella burnetii und Bartonella sp bei einem Kranken mit einem Herzklappenfehler hervorgerufen wird, **dadurch gekennzeichnet, daß** eine Dosierung der Antikörper des Typs IgG, die gegen das Bakterium Coxiella burnetii der Phase I gerichtet sind, und der Antikörper des Typs IgG, die gegen das Bakterium Bartonella sp gerichtet sind an einem selben Serum eines Kranken durchgeführt und bestimmt wird, ob der Titer der Antiköper IgG größer oder gleich 1/800-stel ist.

2. Diagnosemethode nach Anspruch 1, **dadurch gekennzeichnet, daß** eine Dosierung durchgeführt wird, bei der:
1. die beiden genannten Bakterien auf einen festen Träger aufgebracht werden, und
2. die Bakterien mit einem Serum des Kranken, das auf 1/800-stel verdünnt ist und dem ein Antihumanimmunglobulin IgG, das vorzugsweise markiert ist, hinzugefügt wurde, zur Reaktion gebracht werden, und
3. überprüft wird, ob sich die Antihumanimmunglobuline IgG, die vorzugsweise markiert sind, auf dem festen Träger fixieren.

3. Diagnosemethode nach Anspruch 1 oder 2, **dadurch gekennzeichnet, daß** das Bakterium Bartonella sp ein Bakterium Bartonella quintana oder Bartonella henselae ist.

4. Diagnosemethode nach einem der Ansprüche 1 bis 3, **dadurch gekennzeichnet, daß** auf den festen Träger die folgenden Bakterien aufgebracht werden:
- ein Bakterium Coxiella burnetii der Phase I, und
- ein Bakterium Bartonella quintana.

5. Diagnosemethode nach einem der Ansprüche 1 bis 4, **dadurch gekennzeichnet, daß** das Antihumanimmunglobulin IgG eine Markierung umfaßt, die vorzugsweise durch Fluoreszenz erfaßbar ist.

6. Diagnosekoffer für den Einsatz einer Diagnosemethode nach einem der Ansprüche 1 bis 5, **dadurch gekennzeichnet, daß** er umfaßt:
- einen festen Träger, auf dem als für Endokarditis verantwortliche Bakterien nur die Bakterien Coxiella burnetii der Phase I und ein Bakterium Bartonella sp aufgebracht wurden, und
- als Lösung, umfassend ein Antihumanglobulin, das für die Erfassung von gegen diese Bakterien gerichteten Antikörpern eingesetzt wird, eine einzige Lösung, die als Antihumanimmunglobuline nur ein Antihumanimmunglobulin IgG umfaßt.

## Claims

1. *In vitro* serological method for diagnosing infectious endocarditis with negative hemoculture caused by the bacteria *Coxiella burnetii* and *Bartonella sp* in a patient suffering from a heart valve disorder, **characterized in that** an assay is conducted of IgG-type antibodies directed against phase 1 *Coxiella burnetii* bacterium and of IgG-type antibodies directed against the bacterium *Bartonella sp* on one same serum from said patient, and it is determined whether the titer of said IgG antibodies is 1:800 or more.

2. Diagnosis method as in claim 1, **characterized in that** an assay is performed in which:
1. the two said bacteria are deposited on a solid support, and
2. said bacteria are caused to react with a serum from said patient diluted to 1:800, in which an anti-human IgG immunoglobulin has been added, preferably labelled, and
3. it is verified whether said anti-human IgG immunoglobulins, preferably labelled, attach to said solid support.

3. Diagnosis method as in claim 1 or 2, **characterized in that** said *Bartonella sp* bacterium is a *Bartonella quintana* or *Bartonella henselae* bacterium.

4. Diagnosis method as in any of claims 1 to 3, **characterized in that** the following bacteria are deposited on said solid support:
- a phase 1 *Coxiella burnetii* bacterium, and
- a *Bartonella quintana* bacterium.

5. Diagnosis method as in any of claims 1 to 4, **characterized in that** the anti-human IgG immunoglobulin comprises labelling, preferably detectable by fluorescence.

6. Diagnostic kit which can be used to implement a diagnosis method as in any of claims 1 to 5, **characterized in that** it contains:
- a solid support on which, as said bacteria responsible for endocardites, solely phase 1 *Coxiella burnetii* and a *Bartonella sp.* bacterium have been deposited, and
- as solution containing an anti-human immunoglobulin involved in the detection of antibodies directed against said bacteria, a single solution solely containing an anti-IgG immunoglobulin as anti-human immunoglobulin.
